# EUROPEAN PATENT SPECIFICATION

(11) **EP 0 614 372 B1**
(45) Date of publication and mention of the grant of the patent: **29.12.1999**
(21) Application number: 92923380.7
(22) Date of filing: 30.10.1992
(51) Int. Cl.: A61K 39/12, C07K 1/36, C07K 14/435, C07K 14/52, C12N 9/48

(54) **ANTIVIRAL FACTOR FROM CD8+ CELLS**
ANTIVIRALER FAKTOR AUS CD8+ ZELLEN
FACTEUR ANTIVIRAL DES CD8+ CELLULES

(30) Priority: 01.11.1991 US 786114
(43) Date of publication of application: 14.09.1994
(73) Proprietor: THE REGENTS OF THE UNIVERSITY OF CALIFORNIA, Oakland, California 94612-3550 (US)
(72) Inventor: LEVY, Jay, A., San Francisco, CA 94118 (US); MACKEWICZ, Carl, E., San Francisco, CA 94131 (US)
(74) Representative: Tiedtke, Harro, Dipl.-Ing.
(86) International application number: US9209302
(87) International publication number: WO9308835

(56) References cited:
- THE JOURNAL OF CLINICAL INVESTIGATION, vol. 87,no. 4, April 1991 pages 1462-1466, MACKEWICZ C.E. ET AL.
- JOURNAL OF ACQUIRED IMMUNE DEFICIENCY SYNDROMES, vol. 4,no. 5, May 1991 pages 480-487, BRINCHMANN J.E. ET AL.
- J0URNAL OF VIROLOGY, vol. 65,no. 11, November 1991 pages 5921-5927, WALKER C.M. ET AL.
- Journal of Immunology, Volume 144, issued 1990, J.E. BRINCHMANN et al., "CD8+ T Cells Inhibit HIV Replication in Naturally Infected CD4+ T Cells", pages 2961-2966, see Abstract.
- Immunology, Volume 66, issued 1989, C.M. WALKER et al., "A Diffusible Lymphokine Produced by CD8+ T Lymphocytes Suppresses HIV Replication", pages 628-630, see entire document.
- Science, Volume 234, issued 19 December 1986, C.M. WALKER et al., "CD8+ Lymphocytes can Control HIV Infection In Vitro by Suppressing Virus Replication", pages 1563-1566, see entire article.
- Methods in Enzymology, vol.182, p.299, (1990)

## Description

### Field of the invention

This invention provides for a purified composition containing cellular factor designated CD8⁺ cell antiviral factor [CAF]. This cell factor is secreted by activated CD8⁺ T-cells. The factor is suitable for inhibiting the replication of human immunodeficiency virus [HIV] and related retroviruses in infected mammals.

### Information Disclosure

CAF activity has been described in an unpurified and complex medium. The medium has been demonstrated to inhibit HIV replication *in vitro*. No reference known to the inventors has demonstrated that a single, unique and novel cell-secreted factor is responsible for this antiviral activity. Walker, C.M. and Levy, J.A., 1989, A Diffusible Lymphokine Produced by CD8⁺ T Lymphocytes Suppresses HIV Replication, *Immunology*, 66,628-630, 1989; Brinckmann, J.E. et al., CD8⁺ T Cells Inhibit HIV Replication in Naturally Infected CD4⁺ T Cells, *J. Immunology*, 144,2961-2966, 1990; and, Walker, C.M., et al., CD8⁺ Lymphocytes can Control HIV Infection in Vitro by Suppressing Virus Replication, *Science*, 234, 1563-1566, 1986.

Similarly, J.E. Brinchmann et al. *Journal of AIDS*, vol. 4(2), 480-488, 1991, describe cell-free supernatant from CD8⁺ T cells, which has been filtered and subsequently volume-concentrated. HIV replication studies show that activated CD8⁺ T cells secrete a HIV-1-inhibitory soluble factor. Blocking experiments using an IFN-α₂ neutralizing monoclonal antibody showed that this HIV-1-inhibitory factor is not IFN-α₂.

CD8⁺ cell anti-HIV activity has been correlated with a patient's clinical condition. Mackewicz, C.E. et al., CD8⁺ cell anti-HIV activity correlates with the clinical state of the infected individual, *J. Clin. Invest.* 87, 1462-1466, 1991.

### SUMMARY OF THE INVENTION

This invention provides for a purified composition of CD8⁺ cell antiviral factor being released from activated CD8⁺ cells wherein said CAF in said composition is defined by the following properties : cells; biologically inactivated by pH in ranges 10-12; is acid insensitive; is insensitive to ether; is resistant to heat of 56°C for 30 minutes; is sensitive to Leupeptin; is resistant to trypsin treatment and does not bind to antibodies specific for IFNα, IFNβ, IFNγ, TNFβ, IL6, IL4, TGF-β or TNFα. CAF does not affect the activation or proliferation of CD4⁺ cells and inhibits viral replication by interfering with RNA transcription. CAF is not one of the previously described cytokines as determined by biologic, molecular, and serologic assays. These cytokines include those provided in table 1. In addition, this invention provides for a pharmaceutical composition in a unit dosage form consisting of both one or more active ingredients and one or more pharmaceutically acceptable excipients wherein one of the active ingredients is the composition defined by the above characteristics. Excipients are pharmacologically inert ingredients such as sterile water, salts, buffering agents having a pKa in the physiological range of 6.0 to 8.0 and the like. Active ingredients are pharmacologically active compounds such as antibiotics, hormones, immunologic stimulators and the like.

The above factor is useful for treating a mammal infected with a retrovirus such as a lentivirus. Lentiviruses are a member of a group of viruses containing reverse transcriptase. They have complex genomes, characteristic morphology under electron microscopy and generally infect cells of the immune and neurologic systems. The treatment comprises administering an amount of CD8⁺ cell antiviral factor effective to inhibit the replication of the virus. A preferred route of administration is intravenous. The preferred amount administered is between about 1,000 to about 10,000 units per kilogram of body weight of the human. One unit is defined as the concentration of CAF needed to reduce HIV replication in peripheral mononuclear cells by 75% using the assay described herein.

This invention further provides a medical use according to claim 5. This inhibition of the replication of lentivirus can be either *in vitro* or *in vivo*.

### DESCRIPTION OF THE PREFERRED EMBODIMENT

As previously stated, this invention provides for a purified composition containing, CD8⁺ anti-HIV factor [CAF] secreted by peripheral blood mononuclear cells [PMC] expressing the molecule defined as CD8 on their cell surfaces. They are primarily of the activated phenotype, CD8⁺CD11b⁻, CD38⁺ and HLA-DR⁺. CAF is obtained from the CD8⁺ T-cells that have been activated during HIV infection.

CAF is purified from conditioned medium in which activated CD8⁺ cells have been cultured. The CD8⁺ cells are preferably obtained from an individual carrying HIV yet exhibiting no symptoms associated with HIV-induced disease. The cells are withdrawn by standard venipuncture. The PMC are then obtained from anticoagulant blood or buffy coats by gradient centrifugation, e.g., Ficoll/Hypaque.

To separate the CD8⁺ cells from the other PMC, a CD8⁺ specific antibody such as ITI-5C2-/IgM (*J*. *Immunol. Methods*, 90:179) is used to coat the cells. The antibody will specifically bind to the CD8⁺ cells. The CD8⁺ cells are then captured using support-bound antibodies specific for the antibodies attached to the cells. Column chromatography, magnetic beads, or panning on petri dishes are examples of matrices suitable for such cell separations.

The CD8⁺ cells are cultured in any suitable cell most preferably a RPMI based medium with antibiotics to prevent contamination, interleukin 2 [IL2] and with a mitogenic stimulator (e.g., phytohemagglutinin or anti-T-cell receptor (TCR) coated beads for 2-3 days) to promote secretion of CAF. For purification of the factor, cultivation of CD8⁺ cells in any suitable serum-free medium - AIM V (GIBCO) serum-free medium is preferred.

After a suitable incubation (5-13 days), the conditioned cell medium is obtained by either microfiltration or differential centrifugation. The cells can be recovered and refad with fresh medium or replaced. The conditioned medium is then subjected to protein purification procedures designed to eliminate contaminating by-products and concentrate the CAF in each step. Heating inactivates contaminating cellular proteins that might be present and then standard protein purification procedures are used such as molecular sizing, immunospecific affinity or other chromatographic columns.

After the factor is purified to a single band on a polyacrylamide gel or HPLC column (about 90% pure), the purified composition is formulated using standard pharmaceutical excipients (e.g., physiological saline). It can be stored in a lyophilized state or frozen in liquid medium at -20°C or -70°C. The purified composition is administered for injection into the circulatory system. For example it may be dissolved into sterile water, or other aqueous sterile solutions buffered at physiological range of pH 6.4-8.4. The formulation is preferably made isotonic to blood cells. The formulation may also include pharmaceutically active ingredients such as antibiotics, antiviral agents, hormones and the like.

CAF is used to inhibit retrovirus (e.g., lentivirus) replication in mammals such as HIV in humans or feline immunodeficiency virus in cats. A typical patient is treated by injecting between 1,000-10,000 units or CAF containing composition per kilogram of body weight each day until the patient has recovered clinically and the T-cell count returns to normal.

To monitor the purity or effectiveness of the CAF, it is useful to assay for inhibition of replication of HIV in CD4⁺ cells. Cells are either naturally infected (endogenous virus assay) or artificially infected (acute virus infection assay). In the endogenous virus assay, the CD4⁺ cells are obtained from individuals known to be infected with HIV using immuno- selection techniques, for example immuno-magnetic beads. By this process small polystyrene spheres with iron oxide cores coated with monoclonal antibodies to the CD4 or CD8 cell surface protein (Dynal Corporation) are added to a sample of separated (by Ficoll/Hypaque) PMC. After 20-40 minutes of incubation, the CD4⁺ or CD8⁺ cells are removed by a magnetic capture device. The cells are washed and cultured in RPMI-1640 medium supplemented with 10% FBS, 2 mM glutamine, 1% antibiotics (100 units per ml penicillin and 100 µg per ml. streptomycin) and 10 percent human IL2 (Electronucleonics, Silver Spring, Maryland).

Alternatively using the acute virus infection assay, CD4⁺ cells are obtained from uninfected persons and infected with a defined amount of HIV. The cells are then trypsinized to remove input virus and cultured.

In the endogenous virus assay, the CD4⁺ cells are activated using phytohemagglutinin or anti-TCR beads to rapidly divide and produce HIV. Culture fluids containing CAF or purified CAF are assayed for viral magnesium-dependent reverse transcriptase activity at three-day intervals as described in Hoffman, et al., *Virology*, 147, 326 (1985). Alternatively, the replication of virus can be monitored by assaying for other virus specific products such as viral protein p24 as described in *J*. *Immunology*, 144,2961-2966 (1990).

### EXAMPLES

The following examples are presented by way of illustration and not by way of limitation. Those of skill will readily identify numerous ways in which the protocols described below may be modified in non-critical ways.

### Example No. 1. Culturing activated CD8⁺ cells.

CAF is purified from the conditioned medium containing activated CD8⁺ cells. CD8⁺ cells are obtained from the peripheral blood mononuclear cells (PMC) of individuals with HIV infection. PMC are obtained from blood by separation on Ficoll/Hypaque gradients. The cells are washed and cultured in RPMI 1640 medium containing 10% fetal bovine serum (FBS), 1% antibiotics, 10% human IL2. Phytohemagglutinin (3 µg/ml, Sigma) is added at the initiation of a five-milliliter cell culture in a 25 cm³ flask. The cells are permitted to multiply until they reach approximately 6-10x10⁶ cells per milliliter.

Cell cultures of PMC are enriched or depleted of CD8⁺ or CD16⁺ cells using the panning procedures of Wysocki and Sato *PNAS USA*, 75, 2844 (1978). Briefly, plastic-adherent cells (macrophages) are first removed and then 20x10⁶ to 30 x 10⁶ of the non-adherent PMC are incubated in 2 ml of phosphate buffered saline (PBS) containing 10 µg per ml of monoclonal antibodies to either Leu-2a or Leu-11b (Becton-Dickinson) for 20 minutes at room temperature. These antibodies recognize epitopes on the CD8 and CD16 antigens, respectively. The cells are then washed twice, resuspended in 4 mls of PBS containing 1% fetal bovine serum and incubated on a capture plastic petri dish coated with antibody for two hours at 4°C.

The capture plates are prepared using biologic grade petri plates coated with the F(Ab')₂ portion of the goat antibody to mouse immunoglobulin IgG (Tago, Burlingame, California). The plates are coated with a capture antibody (10 µg/ml, in .05 moles Tris, pH 9.5) for 40 minutes at room temperature. Excess antibody is washed off the plate with PBS.

To prevent non-specific attachment of PMC, plates are coated with PBS containing 1% FBS prior to use. After incubation non-adherent cells (that is, the CD8⁻ or CD16⁻fraction) are washed off the plate with cold PBS, and adherent cells (CD8⁺ or CD16⁺) are removed from the plate with a forceful jet of PBS. The Leu 11b monoclonal antibody procedure is performed, if necessary, to remove NK cells from the CD8⁺ cells. The effectiveness of the procedures described are assessed by flow cytometry, as described in J.A. Levy, et al., Clin. *Immuno. Immunopath*., *35*, 328 (1984).

The CD8⁺ enriched cells are then cultured in AIM V medium supplemented with 200 U/ml recombinant IL-2 (Collaborative Research, Bedford, Mass.), 1% antibiotics (100 units per ml penicillin and 100 µgm per ml. streptomycin) and 10 percent human IL2 (Electronucleonics, Silver Spring, Maryland). The cells are allowed to multiply until they reach a concentration of 6-10x10⁶ cells per milliliter. The medium is then separated from the CD8⁺ cells through the use of microfiltration; specifically, a 45 micron Millipore filter. The presence of CAF is monitored using its ability to inhibit HIV replication in susceptible PMC cells.

### Example No. 2. Assaying for CAF activity.

To monitor the purity of the CAF, one assays for the relative inhibition of replication of HIV in CD4⁺ cells using increasingly purified CAF. The cells are obtained from HIV free individuals, washed and cultured in RPMI-1640 medium supplemented with 10% FBS, 2 mM glutamine, 1% antibiotics (100 units per ml penicillin and 100 µg/ml streptomycin) and 10% human IL2 (Electronucleonics, Silver Spring, Maryland). Phytohemagglutinin (Sigma Company, St. Louis, Missouri) is added (3 µg/ml) at the initiation of the culture. After three days, cells in the chamber are washed and refed with fresh IL-2 containing medium described above. The cells are then inoculated with 100 TCID₅₀ (tissue culture infectious doses) of HIV-1. After 1 hour of incubation, the cells are trypsinized to remove input virus. The CAF factor is added and its affect on virus replication monitored every 2-3 days up to 12 days. More specifically, virus replication is assayed for CAF mediated inhibition of HIV replication as measured by magnesium-dependent reverse transcriptase activity as described in Hoffman, et al., *Virology*, 147, 326 (1985) or p24 antigen production. Titration experiments using 90% inhibition of reverse transcriptase activity as a cutoff for effective CAF activity are conducted.

### Example No. 3. Assaying for CAF.

CAF can be identified and distinguished from other cell secreted factors by the following physicochemical assays.

CAF is not sensitive to ether treatment. A 1 ml sample of purified CAF can be exposed to 1 ml. of ethyl ether. After 10 minutes of exposure to the ether at 4°C, the ether (polar) fraction is removed and the ether eliminated by evaporation. This polar fraction is then resuspended in medium. Under these conditions, CAF activity is found in the aqueous solution without decrease in its activity.

CAF is relatively acid insensitive. An aliquote sample of CAF is exposed to a pH in the range of 2-4 or 10-12. After up to 20 hours at these extreme pHs, the CAF is rebuffered to the physiological range of pH 7 and tested for its ability to inhibit reverse transcriptase activity. When exposed to a pH of 10-12, CAF will no longer inhibit HIV activity. CAF activity is unaffected at pHs of 3 and 4 and loses only 10% activity at a pH of 2.

The CAF can be further identified by its relative insensitivity to high heat. An aqueous sample of CAF is be treated for 56° for 30 minutes or at 100°C for 10 minutes. The solution is then cooled and retested for anti-HIV activity. Under these conditions full antiviral affects should be present; however, a reduction of 60% should be seen with the 100° treated material after 30 minutes.

CAF is a protein having selective protease resistance. CAF is insensitive to trypsin activity at 37°C for one hour. Briefly, after exposure to trypsin at 500 µg per ml, soybean trypsin inhibitor is added to the aqueous solution of CAF. CAF is again assayed for its ability to inhibit reverse transcriptase activity. Under these conditions no loss of activity is seen.

Furthermore, CAF is sensitive staph V8 protease beads (Sigma). After CAF is exposed to the beads (1.5 mg/ml supernatant for 2 hr at 37°C), there is a 40-50% activity reduction. As the concentration of beads increases up to 10x, up to 75% of the CAF activity can be eliminated. In addition, protease, type X1A (Protease K) linked to agarose beads (Sigma, up to 30 mg/ml supernatant) showed virtually no effect on CAF activity.

CAF is not any of the commonly known cytokines. Antibodies to alpha, beta or gamma interferon as well as interleukins 4 and 6, TGF-β and tumor necrosis factors α and β will not inhibit *in vitro* anti-HIV activity. In comparison studies, none of the tested cytokines offered the extent of viral inhibition provided by CAF with the exception of α and β interferon and TNFα. CAF is clearly not any of the interleukins 1-10 or 12. (See Table 1).

CAF is inactivated by Leupeptin (1-10 mg/ml) suggesting that CAF could be a protease.

CAF does not bind to ConA columns.

The estimated molecular weight is less than 30 kDa.

CAF is further defined as not affecting the activation or proliferation of CD4⁺ cells and CAF blocks HIV replication at RNA transcription.

**TABLE 1**

| CYTOKINE EFFECT ON HIV REPLICATION IN ACUTELY INFECTED CD4⁺ CELLS | | |
|---|---|---|
| Effect | Cytokine | % Control ∗ ∗ |
| None | IL-1 | 120 |
| | IL-3 | 110 |
| | IL-5 | 100 |
| | IL-6* | 130 |
| | IL-8 | 90 |
| | G-CSF | 80-120 |
| | GM-CSF | 80-120 |
| | IFN-γ* | 90 |
| | IL-10 | 90 |
| | IL-12 | 90 |
| Enhances | IL-2 | 140 |
| | IL-7 | >200 |
| | IL-9 | 160 |
| | TNFα* [<10 µ/ml] | 110-160 |
| Inhibits | IL-4* | 30-90^ |
| | IFN-α* | 0-10 |
| | IFN-β* | 0-10 |
| | TGF-β* | 60-75 |
| | TNFα [≥10 µ/ml] | 30-90 |
| | CD8⁺ cell antiviral factor | 10-40 |
| Results with saturating quantities of the cytokines at the time of peak virus replication. | | |

| | | |
|---|---|---|
| * Antibodies to this cytokine do not block CD8⁺ cell anti-HIV activity. | | |
| ^ Delays HIV replication by 3-6 days. | | |
| ∗ ∗ Percent control is defined as the percent HIV replication in peripheral blood mononuclear cells not cultured with the cytokine (i.e. control cells). | | |

## Claims

1. A purified composition of CD8⁺ cell antiviral factor (CAF) released from activated CD8⁺ cells wherein said CAF in said composition is defined by the following properties:
b. biologically inactivated by pH in ranges of 10-12;
c. acid insensitive;
d. insensitive to ether,
e. resistant to heat of 56 °C for 30 minutes;
f. resistant to trypsin;
g. does not affect activation or proliferation of CD4⁻cells
h. blocks viral replication by inhibiting RNA transcription;
i. sensitive to Leupeptin;
j. does not bind to antibodies specific for IFNα, IFNβ, IFNγ, IL4, IL6, TGF-β, TNFα or TNFβ; and
k. not any of the cytokines listed in Table 1.

2. A pharmaceutical composition in a unit dosage form consisting of both one or more active ingredients and one or more pharmaceutically acceptable excipients wherein one of the active ingredients is the purified composition of claim 1.

3. A composition of claim 2 wherein the excipient comprises sterile water.

4. A composition of claim 3 wherein the excipient further comprises a buffering agent having a pKa of between 8.0 and 6.0.

5. A use of a purified composition according to claim 1 or a pharmaceutical composition according to any one of claims 2 to 4 for the manufacture of a medicament for treating a mammal infected with a retrovirus.

6. The use according to claim 5, wherein the retrovirus is a lentivirus.

7. The use according to claim 6, wherein the lentivirus is human immunodeficiency virus.

8. The use according to claim 5, wherein the mammal is a human.

## Patentansprüche

1. Gereinigte Zusammensetzung des (a) aus aktivierten CD8⁺-Zellen freigesetzten CD8⁺-Zellen-Antivirusfaktors (CAF, *cell antiviral factor*), wobei der CAF in der Zusammensetzung durch die folgenden Eigenschaften definiert ist:
b) biologisch inaktiviert bei einem pH im Bereich von 10 bis 12;
c) Säureunempfindlich;
d) unempfindlich gegenüber Ether;
e) hitzebeständig bei 56 °C für eine Dauer von 30 Minuten;
f) beständig gegenüber Trypsin;
g) beeinflußt nicht die Aktivierung oder Proliferation von CD4⁻-Zellen;
h) blockiert die virale Replikation durch Inhibierung der RNA-Transkription;
i) empfindlich gegenüber Leupeptin;
j) bindet keine Antikörper, die spezifisch für IFNα, IFNβ, IFNγ, IL4, IL6, TGF-β, TNFα oder TNFβ sind; und
k) bindet keine der in Tabelle 1 aufgeführten Cytokine.

2. Pharmazeutische Zusammensetzung in Form einer Dosierungseinheit, die sowohl aus einem oder mehreren aktiven Bestandteilen als auch aus einem oder mehreren pharmazeutisch akzeptablen Arzneimittelträgern besteht, wobei einer der aktiven Bestandteile die gereinigte Zusammensetzung nach Anspruch 1 ist.

3. Zusammensetzung nach Anspruch 2, wobei der Arzneimittelträger steriles Wasser umfaßt.

4. Zusammensetzung nach Anspruch 3, wobei der Arzneimittelträger ferner ein Puffermittel mit einem pKa zwischen 8,0 und 6,0 umfaßt.

5. Verwendung einer gereinigten Zusammensetzung nach Anspruch 1 oder einer pharmazeutischen Zusammensetzung nach einem der Ansprüche 2 bis 4 für die Herstellung eines Medikaments zur Behandlung eines mit einem Retrovirus infizierten Säugetiers.

6. Verwendung nach Anspruch 5, wobei der Retrovirus ein Lentivirus ist.

7. Verwendung nach Anspruch 6, wobei der Lentivirus HIV (humaner Immundefizienzvirus) ist.

8. Verwendung nach Anspruch 5, wobei das Säugetier ein Mensch ist.

## Revendications

1. Composition purifiée du facteur antiviral des cellules CD8⁺ (CAF), libéré de cellules CD8⁺ activées, dans laquelle ledit CAF de ladite composition est défini par les propriétés suivantes :
b. biologiquement inactivé par un pH compris entre 10 et 12 ;
c. insensible aux acides ;
d. insensible à l'éther ;
e. résistant à une chaleur de 56°C pendant 30 minutes ;
f. résistant à la trypsine ;
g. n'affecte pas l'activation ou la prolifération des cellules CD4⁻;
h. bloque la réplication virale par inhibition de la transcription de l'ARN ;
i. sensible à la leupeptine;
j. ne se fixe pas aux anticorps spécifiques de l'IFNα, de l'IFNβ, de l'IFNγ, de l'IL4, de l'IL6, du TGF-β, du TNFα ou du TNFβ ; et
k. n'est pas l'une des cytokines consignées dans le Tableau 1.

2. Composition pharmaceutique sous une forme posologique unitaire, constituée d'un ou plusieurs principes actifs et d'un ou plusieurs excipients acceptables d'un point de vue pharmaceutique, dans laquelle l'un des principes actifs est la composition purifiée de la revendication 1.

3. Composition selon la revendication 2, dans laquelle l'excipient comprend de l'eau stérile.

4. Composition selon la revendication 3, dans laquelle l'excipient comprend en outre un agent tampon ayant un pKa compris entre 8,0 et 6,0.

5. Utilisation d'une composition purifiée selon la revendication 1 ou d'une composition pharmaceutique selon l'une quelconque des revendications 2 à 4, pour préparer un médicament destiné au traitement d'un mammifère infecté par un rétrovirus.

6. Utilisation selon la revendication 5, dans laquelle le rétrovirus est un lentivirus.

7. Utilisation selon la revendication 6, dans laquelle le lentivirus est le virus de l'immunodéficience humaine.

8. Utilisation selon la revendication 5, dans laquelle le mammifère est un humain.
